# EUROPEAN PATENT APPLICATION

(11) **EP 4 353 148 A1**
(43) Date of publication of application: **17.04.2024**
(21) Application number: 22848452.3
(22) Date of filing: 22.07.2022
(51) Int. Cl.: A61B 5/022

(54) **BLOOD PRESSURE MEASURING APPARATUS**

(30) Priority: 30.07.2021 CN 202110869082
(71) Applicant: Huawei Technologies Co., Ltd., Shenzhen, Guangdong 518129 (CN)
(72) Inventor: JIN, Junye, Shenzhen, Guangdong 518129 (CN); YANG, Wenjian, Shenzhen, Guangdong 518129 (CN); ZHAO, Menglong, Shenzhen, Guangdong 518129 (CN); ZHANG, Lei, Shenzhen, Guangdong 518129 (CN); LU, Shiqiang, Shenzhen, Guangdong 518129 (CN); LI, Changming, Shenzhen, Guangdong 518129 (CN)
(74) Representative: Pfenning, Meinig & Partner mbB
(86) International application number: PCT/CN2022/107437
(87) International publication number: WO 2023/005847

(57) **Abstract**

This application provides a blood pressure measuring device. The blood pressure measuring device includes a main body and an airbag. The airbag has an air cavity, and the airbag is fixed to an end of the main body. The main body has a cavity enclosed by a plurality of side walls. An air supply and exhaust apparatus and a first air pressure sensor may be disposed in the cavity of the main body. This is not limited. The air supply and exhaust apparatus includes an air inlet passage and an air outlet passage, and the air supply and exhaust apparatus is communicated with the air cavity of the airbag through a first air passage. The first air pressure sensor includes a first air hole and a second air hole, and a pressure film is provided between the first air hole and the second air hole. In addition, a first vent hole is provided on the side wall of the main body. The first air hole may be communicated with the air cavity of the airbag through a second air passage, and the second air hole may be communicated with the first vent hole through a third air passage. Air pressure on two sides of the pressure film of the first air pressure sensor is air pressure in the airbag and external atmospheric pressure respectively, and is not affected by air pressure in the cavity, so that blood pressure measurement accuracy of the blood pressure measuring device is high.

## Description

### CROSS-REFERENCE TO RELATED APPLICATIONS

This application claims priority to Chinese Patent Application No. 202110869082.3, filed with the China National Intellectual Property Administration on July 30, 2021 and entitled "BLOOD PRESSURE MEASURING DEVICE", which is incorporated herein by reference in its entirety.

### TECHNICAL FIELD

This application relates to the field of electronic device technologies, and in particular, to a blood pressure measuring device.

### BACKGROUND

Nowadays, people pay more attention to health of themselves and their families, and blood pressure measurement is also particularly important. With progress and development of science and technology, in addition to emergence of blood pressure measuring devices for home use, some wearable devices (such as smartwatches or smart bands) also start to be integrated with a blood pressure measuring function, which provides a possibility for a user to measure blood pressure anytime and anywhere.

In current blood pressure measuring devices, a micropump and a pressure sensor are directly disposed inside a main body of the blood pressure measuring device. The pressure sensor may obtain a blood pressure value of a user by measuring air pressure inside the main body of the blood pressure measuring device and air pressure of an airbag. However, in a process of performing blood pressure measurement by using the blood pressure measuring device, the micropump inflates gas inside the main body into the airbag, causing an air pressure fluctuation inside the main body. In this case, the air pressure inside the main body measured by the pressure sensor is unstable. As a result, accuracy of the blood pressure value measured by the blood pressure measuring device is low.

Therefore, how to provide a blood pressure measuring device that can meet blood pressure measurement accuracy has become a problem to be urgently resolved by a person skilled in the art.

### SUMMARY

This application provides a blood pressure measuring device, to reduce impact of internal air pressure of the blood pressure measuring device on blood pressure measurement of the blood pressure measuring device, thereby improving accuracy of the blood pressure measurement.

According to a first aspect, this application provides a blood pressure measuring device. The blood pressure measuring device may include a main body, an airbag, an air supply and exhaust apparatus, and a first air pressure sensor. The main body includes a cavity, and each functional module or component of the blood pressure measuring device may be disposed in the cavity. For example, the foregoing air supply and exhaust apparatus and the first air pressure sensor may be disposed in the cavity. The airbag is fixed to an end of the main body, and the airbag has an air cavity. The air supply and exhaust apparatus includes an air inlet passage and an air outlet passage, and the air supply and exhaust apparatus is communicated with the air cavity of the airbag through a first air passage. The first air pressure sensor includes a first air hole, a second air hole, and a pressure film. The pressure film is located between the first air hole and the second air hole. The first air hole is communicated with the air cavity of the airbag through a second air passage. In addition, the cavity of the main body may be enclosed by a plurality of side walls, and a first vent hole may be provided on the side wall, and the first vent hole is communicated with outside atmosphere. In this way, the second air hole of the first air pressure sensor may be communicated with the first vent hole through a third air passage, so that the first air pressure sensor is communicated with the outside atmosphere through the first vent hole. Using the blood pressure measuring device provided in this application, since air pressure on both sides of the pressure film of the first air pressure sensor are external atmospheric pressure and air pressure in the air cavity of the airbag respectively, the first air pressure sensor is not affected by the air pressure in the cavity in a process of performing blood pressure measurement, blood pressure measurement accuracy of the blood pressure measuring device is high.

Since the air supply and exhaust apparatus is disposed in the cavity of the main body, if both an air inlet passage and an air outlet passage of the air supply and exhaust apparatus are communicated with the cavity of the main body, negative pressure is generated in the cavity of the main body during operation of the air supply and exhaust apparatus (a difference between internal and external air pressure, that is, the air pressure inside the cavity of the main body is less than the external atmospheric pressure). In a conventional blood pressure measuring device, to reduce the negative pressure inside the cavity of the main body, the blood pressure measuring device are not designed to be waterproof. A main reason is that disposing of a waterproof film reduces an air permeating volume of the blood pressure measuring device, so that the negative pressure in the cavity of the main body is large, thereby increasing a system error in the process of blood pressure measurement. In addition, an air pressure value inside the cavity of the main body and fluctuation of the cavity of the main body also cause a blood pressure measurement error. However, most components of the blood pressure measuring device are all disposed in the cavity of the main body. If no waterproof design is performed on the main body, a component in the cavity of the main body of the blood pressure measuring device has a high risk of damage. This affects a service life of the blood pressure measuring device. To resolve this problem, in a possible implementation of this application, a second vent hole may be further provided on the side wall of the main body. The second vent hole is communicated with the outside atmosphere, so that gas of the outside atmosphere can enter the cavity of the main body through the second vent hole, thereby supplementing the air in the cavity of the main body in time to reduce the negative pressure in the cavity.

In addition, to implement a waterproof design of the blood pressure measuring device, a first waterproof ventilation apparatus may be disposed at the second vent hole. To reduce impact of the disposing of the first waterproof ventilation apparatus on the air permeating volume of the second vent hole, the air permeating volume of the first waterproof ventilation apparatus may be greater than or equal to 100 ml/min, so that the gas in the cavity of the main body can be supplemented in time and stably.

However, when the first waterproof ventilation apparatus is blocked, the air permeating volume of the first waterproof ventilation apparatus is greatly reduced, resulting in an increase in the negative pressure in the cavity. In a possible implementation of this application, a blockage condition of the first waterproof ventilation apparatus may be determined by measuring air permeability of the first waterproof ventilation apparatus. During a specific implementation, the blood pressure measuring device may further include a second air pressure sensor. The second air pressure sensor is disposed in the cavity of the main body, and the blood pressure measuring device may determine air permeability of the first waterproof ventilation apparatus based on an air pressure value measured by the second air pressure sensor. The second air pressure sensor may be an absolute pressure sensor. When the air pressure value measured by the second air pressure sensor is greater than a first threshold, it may be determined that the air permeability of the first waterproof ventilation apparatus is good. In this application, the first threshold may be set based on a specific application scenario. For example, the first threshold may be 95 Kpa. In this case, when the air pressure value measured by the second air pressure sensor is greater than 95 Kpa, it is determined that the air permeability of the first waterproof ventilation apparatus is good.

Correspondingly, in some possible implementations, when the air pressure value measured by the second air pressure sensor is lower than the first threshold, it may be determined that the first waterproof ventilation apparatus is blocked.

It may be understood that in this application, a second waterproof ventilation apparatus may be further disposed at the first vent hole, to further improve waterproofness of the blood pressure measuring device.

It can be learned from foregoing introductions of this application that one of the reasons for generating the negative pressure in the cavity of the main body is that the airbag is inflated and deflated by the air supply and exhaust apparatus. To reduce impact of operation of the air supply and exhaust apparatus on the negative pressure in the cavity of the main body, in a possible implementation of this application, a fourth vent hole and a fifth vent hole may be further provided, and an air inlet passage of the air supply and exhaust apparatus may be communicated with the outside atmosphere through the fourth vent hole. The air outlet passage may be communicated with the outside atmosphere through the fifth vent hole. In this way, the air supply and exhaust apparatus may inflate gas in the outside atmosphere into the airbag through the fourth vent hole and the air inlet passage, and exhaust gas in the airbag to the outside atmosphere through the air outlet passage and the fifth vent hole.

In this application, the air supply and exhaust apparatus and the first air pressure sensor may be directly connected to or indirectly connected to the airbag through a corresponding air passage. For example, in a possible implementation of this application, the blood pressure measuring device may further include an air passage cavity, and the air passage cavity is disposed in the cavity of the main body. In addition, the air supply and exhaust apparatus may be communicated with the air passage cavity through the first air passage. The first air hole of the first air pressure sensor may be communicated with the air passage cavity through the second air passage, and the air passage cavity is communicated with the air cavity of the airbag through a fourth air passage. In this way, air passages that are of the air supply and exhaust apparatus and the first air pressure sensor and that are configured to be communicated with the airbag may be first combined through the air passage cavity, and then communicated with the airbag through one air passage. In this case, only one through hole for connecting to the airbag needs to be provided on the side wall of the main body, so that a quantity of openings on the main body can be reduced, to improve waterproofness and structural stability of the blood pressure measuring device.

In a possible implementation of this application, to connect the airbag to the main body, a connection hole may be provided at an end of the main body. Further, the airbag has an air nozzle protruding from a side surface of the airbag in a direction toward the main body. In this way, the air nozzle may be inserted into the connection hole, and the fourth air passage is connected to the air nozzle, to implement communication between the airbag and the air passage cavity. Similarly, in some other possible implementations, the air nozzle may be further disposed at the end of the main body, and the connection hole is provided on the airbag. In this way, connection between the air nozzle and the main body may also be implemented by inserting the air nozzle and the connection hole.

It should be noted that in this application, the airbag may be detachably connected to the main body. In this way, the airbag may be detached or replaced as required. In addition, in a possible implementation of this application, the blood pressure measuring device may further include a photoplethysmography PPG module and an ECG measurement module. The PPG module and the ECG measurement module may be disposed on a bottom surface of the main body. The airbag may also be fixedly connected to an end of the bottom surface of the main body, so that the blood pressure measuring device integrates a plurality of measurement functions and makes a structure of the blood pressure measuring device compact.

To further improve blood pressure measurement accuracy of the blood pressure measuring device, in this application, a calibration apparatus may be further disposed for the first air pressure sensor. In a possible implementation of this application, the blood pressure measuring device may further include a third air pressure sensor. The third air pressure sensor includes a third air hole and a fourth air hole. In addition, a third vent hole is further provided on the side wall of the main body. The third vent hole is communicated with the outside atmosphere. The third air hole of the third air pressure sensor is communicated with the air passage cavity through a seventh air passage, and the fourth air hole is communicated with the third vent hole through an eighth air passage. In this way, the third air pressure sensor may measure an air pressure difference between the outside atmosphere and the air pressure in the air cavity of the airbag.

In addition, because the first air pressure sensor also measures the air pressure difference between the outside atmosphere and the air pressure in the air cavity of the airbag, a measured value of the first air pressure sensor can be calibrated by comparing air pressure differences respectively measured by the first air pressure sensor and the third air pressure sensor. During a specific implementation, when a difference between a pressure difference measured by the first air pressure sensor and a pressure difference measured by the third air pressure sensor falls within a first threshold range, it is determined that the pressure difference measured by the first air pressure sensor is accurate. In this application, the first threshold range may be set based on a specific application scenario. For example, the first threshold range may be -200 pa to 200 pa.

Similarly, when a difference between a pressure difference measured by the first air pressure sensor and a pressure difference measured by the third air pressure sensor falls outside the first threshold range, it is determined that the pressure difference measured by the first air pressure sensor is inaccurate.

In addition to the foregoing configuration, in a possible implementation of this application, the third air pressure sensor configured to calibrate the measured value of the first air pressure sensor may further be configured as an absolute air pressure sensor. In this implementation, the third air pressure sensor includes only one third air hole. The third air hole may be communicated with the air passage cavity through the seventh air passage. A process of calibrating the measured value of the first air pressure sensor through the third air pressure sensor is: When a difference between air pressure in the airbag measured by the first air pressure sensor and air pressure in the airbag measured by the third air pressure sensor falls within a first threshold range, it is determined that the air pressure in the airbag measured by the first air pressure sensor is accurate; and/or, when a difference between air pressure in the airbag measured by the first air pressure sensor and air pressure in the airbag measured by the third air pressure sensor falls outside the first threshold range, it is determined that the air pressure in the airbag measured by the first air pressure sensor is inaccurate. Using this implementation, a quantity of vent holes provided on the main body can be reduced, thereby improving waterproofness of the blood pressure measuring device.

In this application, to reduce impact of an air flow fluctuation in each air passage on measurement accuracy, a buffer structure may be disposed in a pipe of the air passage. For example, a plurality of protrusions may be provided in a pipe that is of the second air passage and that is connected to the first air pressure sensor and the airbag. The plurality of protrusions are alternately arranged at intervals along an extension direction of the pipe. The plurality of protrusions may play a buffering role in a process in which the gas flow in the second air passage, thereby reducing the gas flow fluctuation, and improving measurement accuracy of the first air pressure sensor.

According to a second aspect, this application further provides a blood pressure measuring device. The blood pressure measuring device may include a main body, an airbag, an air supply and exhaust apparatus, a first air pressure sensor, and a second air pressure sensor. The main body includes a cavity, and each functional module or component of the blood pressure measuring device may be disposed in the cavity. For example, the foregoing air supply and exhaust apparatus, the first air pressure sensor, and the second air pressure sensor may be disposed in the cavity. The airbag is fixed to an end of the main body, and the airbag has an air cavity. The air supply and exhaust apparatus includes an air inlet passage and an air outlet passage, and the air supply and exhaust apparatus is communicated with the air cavity of the airbag through a first air passage. The first air pressure sensor includes a first air hole, a second air hole, and a pressure film. The pressure film is located between the first air hole and the second air hole. The first air hole is communicated with the air cavity of the airbag through a second air passage. The second air hole is communicated with the cavity. A pressure difference measured by the first air pressure sensor is determined by air pressure in the cavity of the main body and air pressure in the air cavity of the airbag. In addition, the second air pressure sensor is an absolute air pressure sensor, the second air pressure sensor includes a fifth air hole, and the fifth air hole faces to the second air hole.

Using the blood pressure measuring device provided in this application, the second air pressure sensor is disposed in the cavity of the main body, so that the blood pressure measuring device obtains an air pressure value in the air cavity of the airbag based on external atmospheric pressure, an air pressure value measured by the second air pressure sensor, and a pressure difference measured by the first air pressure sensor. In addition, a difference between the external atmospheric pressure and the air pressure value measured by the second air pressure sensor may be used as an error value for calculating the air pressure in the air cavity of the airbag, and a real air pressure value in the airbag may be obtained, thereby effectively improving blood pressure measurement accuracy of the blood pressure measuring device.

In a possible implementation of this application, the fifth air hole and the second air hole may be alternatively arranged coaxially. In addition, a distance between the fifth air hole and the second air hole may be less than or equal to 1 mm. Since the second air pressure sensor includes only one fifth air hole, the second air pressure sensor can measure the air pressure in the cavity of the main body. In addition, since the distance between the fifth air hole and the second air hole is small, air pressure measured on a side of the fifth air hole may be considered equal to air pressure measured on a side of the second air hole side.

In a possible implementation of this application, a vent hole is further provided on the side wall of the main body. A waterproof ventilation apparatus is disposed at the vent hole. In this case, the blood pressure measuring device may further determine air permeability of the waterproof ventilation apparatus based on the air pressure value measured by the second air pressure sensor. During a specific implementation, when the air pressure value measured by the second air pressure sensor is greater than a first threshold, it can be determined that air permeability of the waterproof ventilation apparatus is good. In this application, the first threshold may be set based on a specific application scenario. For example, the first threshold may be 95 Kpa. In this case, when the air pressure value measured by the second air pressure sensor is greater than 95 Kpa, it is determined that the air permeability of the waterproof ventilation apparatus is good. Correspondingly, in some possible implementations, when the air pressure value measured by the second air pressure sensor is lower than the first threshold, it may be determined that the waterproof ventilation apparatus is blocked. By measuring the air permeability of the waterproof ventilation apparatus, the waterproof ventilation apparatus can be replaced or cleaned in time when the waterproof ventilation apparatus is blocked, to reduce negative pressure in the cavity of the main body, thereby improving measurement accuracy of the blood pressure measuring device.

According to a third aspect, this application further provides a blood pressure measuring device. The blood pressure measuring device may include a main body, an airbag, an air supply and exhaust apparatus, a first air pressure sensor, a second air pressure sensor, and a waterproof ventilation apparatus. The main body includes a cavity, and each functional module or component of the blood pressure measuring device may be disposed in the cavity. For example, the foregoing air supply and exhaust apparatus, the first air pressure sensor, and the second air pressure sensor may be disposed in the cavity. In addition, the cavity is enclosed by a plurality of side walls, and a vent hole is provided on the side wall. The waterproof ventilation apparatus covers the vent hole. The airbag is fixed to an end of the main body, and the airbag has an air cavity. The air supply and exhaust apparatus includes an air inlet passage and an air outlet passage, and the air supply and exhaust apparatus is communicated with the air cavity of the airbag through a first air passage. The first air pressure sensor is configured to measure air pressure in the air cavity of the airbag. The second air pressure sensor is an absolute air pressure sensor. When an air pressure value measured by the second air pressure sensor is greater than a first threshold, it is determined that air permeability of the waterproof ventilation apparatus is good; and/or when the air pressure value measured by the second air pressure sensor is lower than the first threshold, it is determined that the waterproof ventilation apparatus is blocked.

Using the blood pressure measuring device provided in this application, the second air pressure sensor is disposed in the cavity of the main body, so that air permeability of the waterproof ventilation apparatus can be measured, and the waterproof ventilation apparatus can be replaced or cleaned in time, to ensure security of the blood pressure measuring device and stability of blood pressure measurement. Therefore, a blood pressure value measured by the blood pressure measuring device is accurate.

### BRIEF DESCRIPTION OF DRAWINGS

FIG. 1 is a schematic diagram of a structure of a blood pressure measuring device according to an embodiment of this application;
FIG. 2 is a schematic diagram of a framework structure of an existing blood pressure measuring device according to an embodiment of this application;
FIG. 3 is a schematic diagram of a framework structure of a blood pressure measuring device according to an embodiment of this application;
FIG. 4a to FIG. 4c are schematic diagrams of an internal structure of an air passage according to an embodiment of this application;
FIG. 5 is a schematic diagram of a framework structure of a blood pressure measuring device according to another embodiment of this application;
FIG. 6 is a schematic diagram of a framework structure of a blood pressure measuring device according to another embodiment of this application;
FIG. 7 is a schematic diagram of an explosion structure of a waterproof ventilation apparatus according to an embodiment of this application;
FIG. 8 is a schematic diagram of a framework structure of a blood pressure measuring device according to another embodiment of this application;
FIG. 9 is a schematic diagram of a local structure of a blood pressure measuring device according to an embodiment of this application;
FIG. 10 is a schematic diagram of a structure of an airbag according to an embodiment of this application;
FIG. 11 is an enlarged view of a local structure at A in FIG. 10;
FIG. 12 is a schematic diagram of a framework structure of a blood pressure measuring device according to another embodiment of this application;
FIG. 13 is a schematic diagram of a framework structure of a blood pressure measuring device according to another embodiment of this application;
FIG. 14 is a schematic diagram of a framework structure of a blood pressure measuring device according to another embodiment of this application; and
FIG. 15 is a schematic diagram of a framework structure of a blood pressure measuring device according to another embodiment of this application.

Reference numerals:
1: main body; 101: cavity; 102a, 102b, 102c, 102d, 102e: vent hole; 103: connection hole; 104: PPG module;
105: ECG measurement module; 2: airbag; 201: air nozzle; 2011: clamping structure; 3: wrist strap; 4: air supply and exhaust apparatus;
401: air inlet passage; 402: air outlet passage; 5a: first air pressure sensor; 501: first air hole; 502: second air hole;
5b: second air pressure sensor; 505: fifth air hole; 5c: third air pressure sensor; 503: third air hole; 504: fourth air hole;
61: first air passage; 62: second air passage; 63: third air passage; 64: fourth air passage; 65: fifth air passage; 66: sixth air passage;
67: seventh air passage; 601: pipe; 602: protrusion; 7: driving apparatus; 8: first waterproof ventilation apparatus; 801: foam;
802: balance hole steel sheet; 803: PET layer; 804: waterproof membrane; 805: double-sided tape; 806: waterproof glue;
9: second waterproof ventilation apparatus; 10: air passage cavity; 11: air valve; 12: third waterproof ventilation apparatus.

### DESCRIPTION OF EMBODIMENTS

To make the objectives, technical solutions, and advantages of this application clearer, the following further describes this application in detail with reference to the accompanying drawings.

To facilitate understanding of the blood pressure measuring device provided in embodiments of this application, the following first describes an application scenario of the blood pressure measuring device. The blood pressure measuring device may be, but is not limited to, a large-volume device configured to measure blood pressure, such as a medical device or a household device, or may be a portable electronic device with a blood pressure measuring function, such as a smartwatch or a smart band. A smartwatch is used as an example. The smartwatch may be worn on a wrist of a user, so that a physical sign such as blood pressure of the user can be measured at any time, to predict a physical condition, thereby effectively avoiding a dangerous secondary disease such as stroke caused by hypertension.

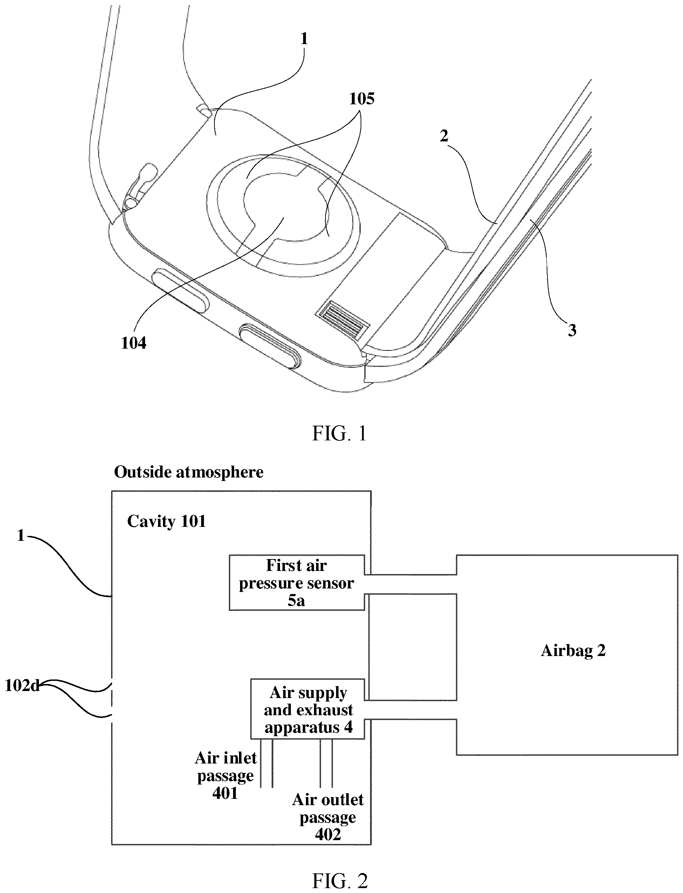

FIG. 1 is a schematic diagram of a structure of a smartwatch with a blood pressure measuring function according to an embodiment of this application. A blood pressure measuring device with a blood pressure measuring function may generally include a main body 1 and an airbag 2. The airbag 2 may be fixed to an end of the main body 1. For example, the airbag 2 may be fixed to an end face of a bottom surface of the main body 1. In this application, the bottom surface of the main body 1 is a surface that is of the main body 1 and that is in direct contact with a wrist when the smartwatch is worn on the wrist. In addition, the blood pressure measuring device may further include a wrist strap 3. As shown in FIG. 1, the airbag 2 may be located on a side of the wrist strap 3 that faces the wrist of the user. In this way, when the wrist strap 3 is wound around the wrist of the user, the airbag 2 may be pressed against the wrist, and the airbag 2 is attached to the wrist, thereby facilitating measurement of the user's blood pressure. It may be understood that the airbag 2 and the wrist strap 3 may be fixed, but not limited to, by clamping, bonding, riveting, or the like, to reduce friction generated by mutual movement between the airbag 2 and the wrist strap 3, thereby reducing a risk of airbag wear, to improve a service life of the blood pressure measuring device.

In addition to the foregoing structure, a photoplethysmography (photoplethysmography, PPG) module may generally be further disposed in the smartwatch with the blood pressure measuring function. The PPG module 104 may be alternatively disposed on the bottom surface of the main body 1. In addition, the PPG module 104 may be alternatively disposed on a middle region of the bottom surface of the main body 1 (with reference to a middle circular region of the bottom surface of the main body 1 shown in FIG. 1), to improve measuring accuracy of the PPG module 104. Since the PPG module 104 may implement continuous measurement of a heart rate value of a human body, a function of performing a single blood pressure measurement by using the airbag 2 may be integrated with a continuous heart rate measurement function of the PPG module 104 by disposing the airbag 2 and the PPG module 104 on the smartwatch simultaneously. A problem of continuous blood pressure measurement is resolved by accurate algorithm calculation.

Still refer to FIG. 1. An electrocardiogram (electrocardiogram, ECG) measurement module may be further disposed in the smartwatch in this embodiment of this application. The ECG measurement module 105 may be alternatively disposed on the bottom surface of the main body 1. In addition, the ECG measurement module 105 may be alternatively disposed in the middle region of the main body 1. For example, the ECG measurement module 105 may be disposed on a peripheral side of the PPG module 104, (with reference to two arc-shaped regions in the middle of the bottom surface of the main body 1 shown in FIG. 1), thereby implementing an electrocardiogram measuring function of the smartwatch.

FIG. 2 is a schematic diagram of a framework structure of a conventional blood pressure measuring device. The main body 1 has a cavity 101. A main functional module and a component (for example, a circuit component such as a processor and a sensor) of the blood pressure measuring device may be disposed in the cavity 101 of the main body 1, for example, an air supply and exhaust apparatus 4 and a first air pressure sensor 5a. An end of the airbag 2 may be communicated with the air supply and exhaust apparatus 4 and the first air pressure sensor 5a through an air nozzle, and the airbag 2 may be wound around a wrist of a user. When the blood pressure measuring device is used for blood pressure measurement, the airbag 2 may be inflated and deflated by the air supply and exhaust apparatus 4. The first air pressure sensor 5a may measure air pressure in the cavity 101 of the main body 1 and air pressure in the airbag 2 during the inflation and deflation process. A blood pressure value of the user may be obtained by using an algorithm based on the two air pressure values.

It can be understood from introductions of the blood pressure measuring process of the blood pressure measuring device that since the air supply and exhaust apparatus 4 is disposed in the cavity 101 of the main body 1, in the process of inflating the airbag 2 by the air supply and exhaust apparatus 4, the air in the cavity 101 of the main body 1 is inflated into the airbag 2. In this way, the air pressure in the cavity 101 of the main body 1 is less than atmospheric pressure outside the main body 1, so that there is an air pressure difference between the cavity 101 of the main body 1 and the outside of the main body 1, or it may be understood that negative pressure is generated in the cavity 101 of the main body 1. The existence of the negative pressure may cause an error in the blood pressure measuring process. In addition, in the process of inflating and deflating the airbag 2 by the air supply and exhaust apparatus 4, the air pressure fluctuation in the cavity 101 of the main body 1 is caused, and the air pressure fluctuation also causes an error in the blood pressure measurement.

Based on this, this embodiment of this application provides a blood pressure measuring device, to reduce impact of the air pressure in the cavity 101 of the main body 1 of the blood pressure measuring device on blood pressure measurement, thereby improving accuracy of the blood pressure measurement. For ease of understanding, in the following embodiments of this application, a smartwatch is used as an example to describe a specific structure of the blood pressure measuring device in detail.

Terms used in the following embodiments are merely intended to describe specific embodiments, and are not intended to limit this application. As used in the specification and the appended claims of this application, the singular expressions "a/an", "one", "the foregoing", "the" and "this" are intended to also include such expressions as "one or more", unless otherwise clearly indicated in the context. It should be further understood that in the following embodiments of this application, "at least one" and "one or more" mean one, two, or more. The term "and/or" is used to describe an association relationship between associated objects and indicates that three relationships may exist. For example, A and/or B may represent: Only A exists, both A and B exist, and only B exists, where A and B may be singular or plural. The character "/" generally represents that the associated object is in an "or" relationship.

Reference to "an embodiment", "some embodiments", or the like described in the specification of this application indicates that one or more embodiments of this application include a specific feature, structure, or characteristic described with reference to embodiments. Therefore, statements "in one embodiment", "in some embodiments", "in some other embodiments", "in some another embodiment" and the like that differ in this specification do not necessarily refer to a same embodiment, but rather means "one or more but not all embodiments", unless otherwise specified. The terms "include", "comprise", "have", and variations thereof all mean "including, but not limited to", unless otherwise particularly specified.

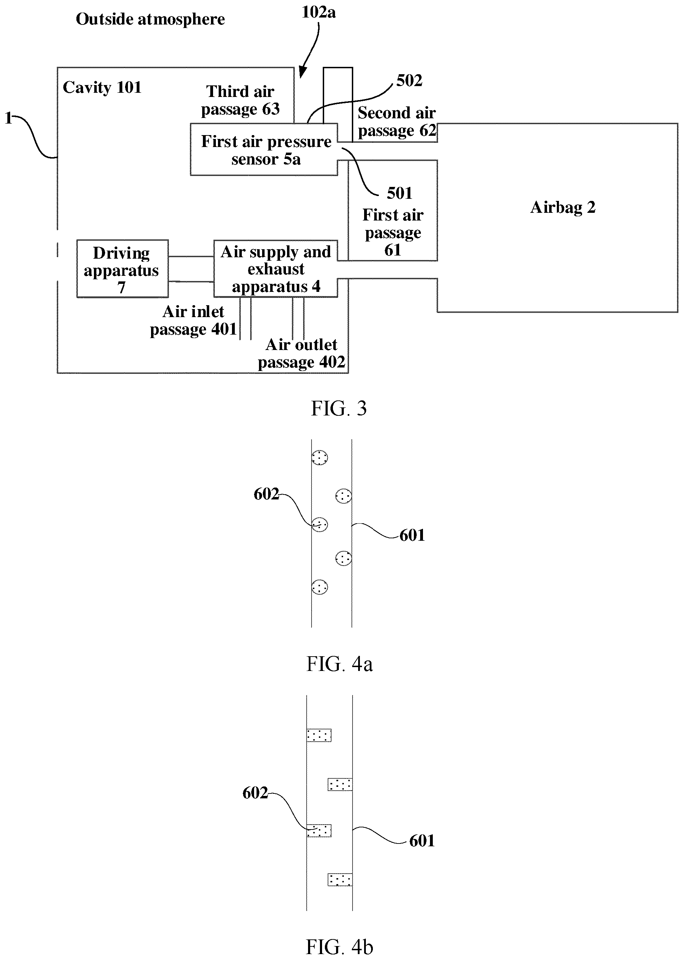

FIG. 3 is a schematic diagram of a framework structure of a blood pressure measuring device according to an embodiment of this application. In this embodiment of this application, the blood pressure measuring device may include a main body 1 and an airbag 2. The main body 1 has a plurality of side walls, and the plurality of side walls are connected to each other, to enclose a cavity 101 of the main body 1. A main functional module and component of the blood pressure measuring device may be disposed in the cavity 101 of the main body 1. The airbag 2 may be fixed to one side wall of the main body 1, and the airbag 2 has an air cavity.

Still refer to FIG. 3. In this embodiment of this application, the blood pressure measuring device may further include an air supply and exhaust apparatus 4 and a first air pressure sensor 5a. The air supply and exhaust apparatus 4 and the first air pressure sensor 5a are disposed in the cavity 101 of the main body 1. The air supply and exhaust apparatus 4 includes an air inlet passage 401 and an air outlet passage 402. Both the air inlet passage 401 and the air outlet passage 402 are communicated with the cavity 101 of the main body 1. In addition, the air supply and exhaust apparatus 4 is communicated with the air cavity of the airbag 2 through a first air passage 61. In this way, air in the cavity 101 of the main body 1 may enter the air supply and exhaust apparatus 4 through the air inlet passage 401, and enter the airbag 2 through the first air passage 61, thereby implementing inflation of the airbag 2 by the air supply and exhaust apparatus 4. On the contrary, when the gas in the airbag 2 needs to be exhausted, the air supply and exhaust apparatus 4 extracts the gas in the airbag 2 through the first air passage 61, and exhausts the air to the cavity 101 of the main body 1 through the air outlet passage 402.

In addition, since the air inlet passage 401 and the air outlet passage 402 of the air supply and exhaust apparatus 4 do not work simultaneously, in a possible embodiment of this application, the air inlet passage 401 and the air outlet passage 402 may be merged. To be specific, only one air passage is disposed on the air supply and exhaust apparatus 4, the air supply and exhaust apparatus 4 may inflate the airbag 2 through the air passage, and the gas in the airbag 2 may be extracted through the air passage, thereby simplifying a structure of the blood pressure measuring device.

In this application, a specific structure of the air supply and exhaust apparatus 4 is not limited. For example, the air supply and exhaust apparatus 4 may be an air pump, and a volume of the air pump may be set based on a requirement of the airbag 2 of the blood pressure measuring apparatus for air supply and exhaust and a size of the cavity 101 of the main body 1. In addition, considering that a volume of the main body 1 of the smartwatch with a blood pressure measuring function is small, so that space of the cavity 101 of the smartwatch is also small. Therefore, a volume of the air pump disposed in the smartwatch is also small. In some possible embodiments of this application, the air pump may be fixed to a mechanical part (not shown), and then the air pump is mounted on the main body 1 by fixing the mechanical part to the main body 1. A material of the mechanical part may be, but is not limited to, metal or non-metal with high strength, to enable the mechanical part can reliably support the air pump, thereby improving structural reliability of the air pump. In this application, a manner of fixing the air pump and the mechanical part is not limited. For example, the air pump and the mechanical part may be fixedly connected through glue dispensing, threaded connection, or the like. In addition, in some embodiments of this application, the glue dispensing may be further performed around the air pump, to seal the air pump and improve structural stability of the air pump.

Still refer to FIG. 3. The blood pressure measuring device may further include a driving apparatus 7. The driving apparatus 7 is electrically connected to the air supply and exhaust apparatus 4, and the driving apparatus 7 may be configured to provide driving force for an inflation and deflation process of the air supply and exhaust apparatus 4. In this application, the driving apparatus 7 is not specifically limited. For example, the driving apparatus 7 may be a motor or the like.

When the first air pressure sensor 5a is specifically disposed, the first air pressure sensor 5a may be a differential air pressure sensor. Further, the first air pressure sensor 5a has a first air hole 501 and a second air hole 502. Specific disposing positions of the first air hole 501 and the second air hole 502 on the first air pressure sensor 5a are not limited. For example, the first air hole 501 and the second air hole 502 may be provided on two end faces disposed opposite to each other of the first air pressure sensor 5a. In addition, the first air pressure sensor 5a may further include a pressure film (not shown). The pressure film is disposed between the first air hole 501 and the second air hole 502. When air pressure at the first air hole 501 and the second air hole 502 is different, the pressure film generates a deformation. The first air pressure sensor 5a may determine an air pressure difference between a side of the first air hole 501 and a side of the second air hole 502 based on a size and direction of the deformation caused by the pressure film.

In this embodiment shown in FIG. 3, the first air hole 501 of the first air pressure sensor 5a may be communicated with the air cavity of the airbag 2 through a second air passage 62. In addition, a vent hole 102a is provided on the side wall of the main body 1, and the vent hole 102a is communicated with the outside atmosphere. The second air hole 502 of the first air pressure sensor 5a is communicated with the vent hole 102a through a third air passage 63, thereby implementing communication between the second air hole 502 and the outside atmosphere. In this application, a specific disposing position of the vent hole 102a is not limited. The vent hole 102a may be adjusted based on a structural design of the main body 1 and a specific disposing position of the first air pressure sensor 5a. In addition, a specific shape of the vent hole 102a is not limited in this application. For example, the vent hole 102a may be a hole in a regular shape, such as a round hole, an elliptical hole, or a square hole, or may be a hole in some possible irregular shapes.

Therefore, in this application, air pressure in a side of the first air hole 501 of the pressure film of the first air pressure sensor 5a is air pressure in the airbag 2, and air pressure in a side of the second air hole 502 of the pressure film is the atmospheric pressure. Therefore, the first air pressure sensor 5a is only configured to measure an air pressure difference between the air pressure in the airbag 2 and the atmospheric pressure. However, it is generally considered that the atmospheric pressure is constant, and a blood pressure value measured by using the blood pressure measuring device provided in this application is accurate. In addition, since the air pressure difference measured by the first air pressure sensor 5a is not affected by air pressure in the cavity 101 of the main body 1, accuracy of blood pressure measurement of the blood pressure measuring device is further improved.

In each embodiment of this application, a specific disposing form of each air passage is not limited. The air passage may be disposed in a straight line, or may be a curved device. Specifically, an adaptive adjustment may be performed based on a component in internal space of the blood pressure measuring device. It may be understood that for clarity of the illustrations, in each schematic diagram of this application, each air passage is shown as a straight line.

In addition, considering that gas in each air passage (for example, the first air passage 61, the second air passage 62, the third air passage 63) may have a problem of an airflow fluctuation during flowing, and the airflow fluctuation may cause an air pressure fluctuation, thereby affecting a result of blood pressure measurement. To resolve this problem, FIG. 4a is a schematic diagram of an internal structure of an air passage according to an embodiment of this application. In this embodiment shown in FIG. 4a, a plurality of protrusions 602 are disposed on an inner wall of a pipe 601 of the air passage. The plurality of protrusions 602 are alternately arranged at intervals and along an extension direction of the pipe 601, to improve air pressure fluctuation noise caused by airflow fluctuation, thereby achieving a purpose of improving accuracy of blood pressure measurement.

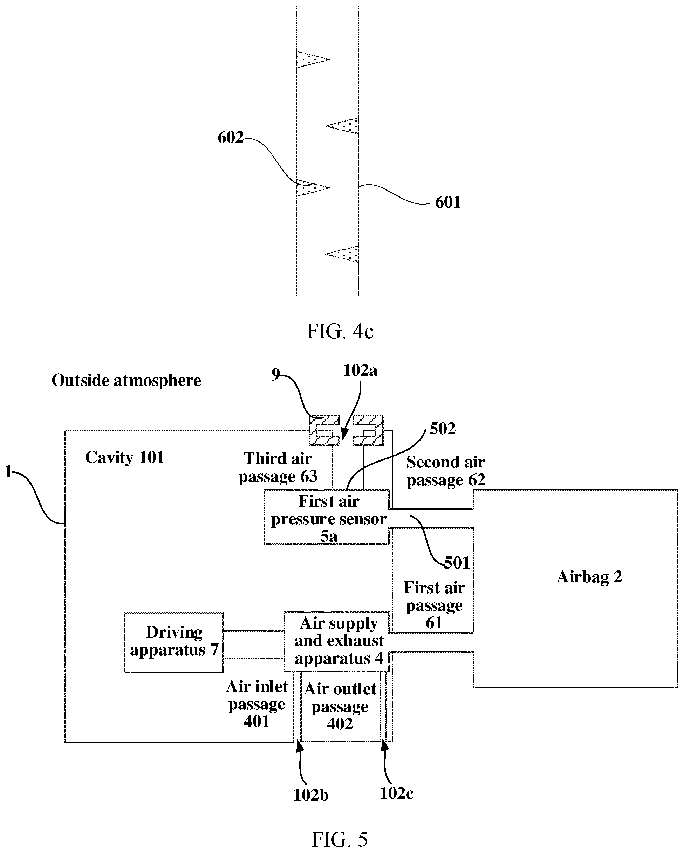

In this application, a specific disposing form of the protrusion 602 is not limited. For example, a cross-sectional shape of the protrusion 602 may be a circle shown in FIG. 4a, a rectangle shown in FIG. 4b, or a regular shape such as a triangle shown in FIG. 4c. In some other embodiments of this application, the protrusion 602 may alternatively be some possible irregular shapes, which are not listed one by one here. However, it should be understood as falling within the protection scope of this application.

Based on introductions in the foregoing embodiment of this application, the first air pressure sensor 5a is communicated with the vent hole 102a on the side wall of the main body 1 through the second air passage 62, to enable the second air hole 502 of the first air pressure sensor 5a to be communicated with the outside atmosphere. In some possible embodiments of this application, both the air inlet passage 401 and the air outlet passage 402 of the air supply and exhaust apparatus 4 may directly communicate with the outside atmosphere through the vent hole provided on the side wall of the main body 1. FIG. 5 is a schematic diagram of a structure of a blood pressure measuring device according to another embodiment of this application. In this embodiment, a vent hole 102b and a vent hole 102c may further be provided on a side wall of the main body 1. The air inlet passage 401 of the air supply and exhaust apparatus 4 is communicated with the outside atmosphere through the vent hole 102b. The air exhaust channel 402 is communicated with the outside atmosphere through the vent hole 102c. In this way, during operation of the air supply and exhaust apparatus 4, air in the outside atmosphere may be inflated into the airbag 2 through the air inlet passage 401 and the first air passage 61. In addition, when the gas in the airbag 2 needs to be exhausted, the air supply and exhaust apparatus 4 extracts the gas in the airbag 2 through the first air passage 61, and exhausts the air into the outside atmosphere through the air outlet passage 402. During the process, the air pressure in the cavity 101 of the main body 1 is not affected by the operation of the air supply and exhaust apparatus 4, and can be maintained in a stable state. Using the blood pressure measuring device provided in this embodiment, impact of the air pressure in the cavity 101 of the main body 1 on normal operation of the air supply and exhaust apparatus 4 can be avoided, thereby effectively improving security and working reliability of the blood pressure measuring device.

To ensure a normal operation of the air supply and exhaust apparatus 4, in addition to the foregoing mentioned configuration in which both the air inlet passage 401 and the air outlet passage 402 directly communicate with the outside atmosphere through the vent hole on the side wall of the main body 1, this may be alternatively achieved by reducing negative pressure in the cavity 101 of the main body 1. In this application, reducing the negative pressure in the cavity 101 of the main body 1 means reducing an air pressure difference between the gas in the cavity 101 and the outside atmosphere outside the main body 1.

To reduce the negative pressure in the cavity 101 of the main body 1, a vent hole 102d is usually provided on the side wall of the main body 1 by a conventional blood pressure measuring device, and a waterproof design is not performed at the vent hole 102d. Still refer to FIG. 2. In this way, the air in the cavity 101 of the main body 1 may be supplemented in time, thereby ensuring accuracy of the blood pressure measurement.

However, most functional modules and components of the blood pressure measuring device are all disposed in the cavity 101 of the main body 1. If no waterproof design is performed on the main body 1, the functional modules and components in the cavity 101 of the main body 1 of the blood pressure measuring device have a high risk of damage. This affects a service life of the blood pressure measuring device. Especially for a portable electronic device that is frequently worn by a user and has a large quantity of application scenarios, such as a smartwatch or a smart band, a necessity of a waterproof design of the portable electronic device is more significant.

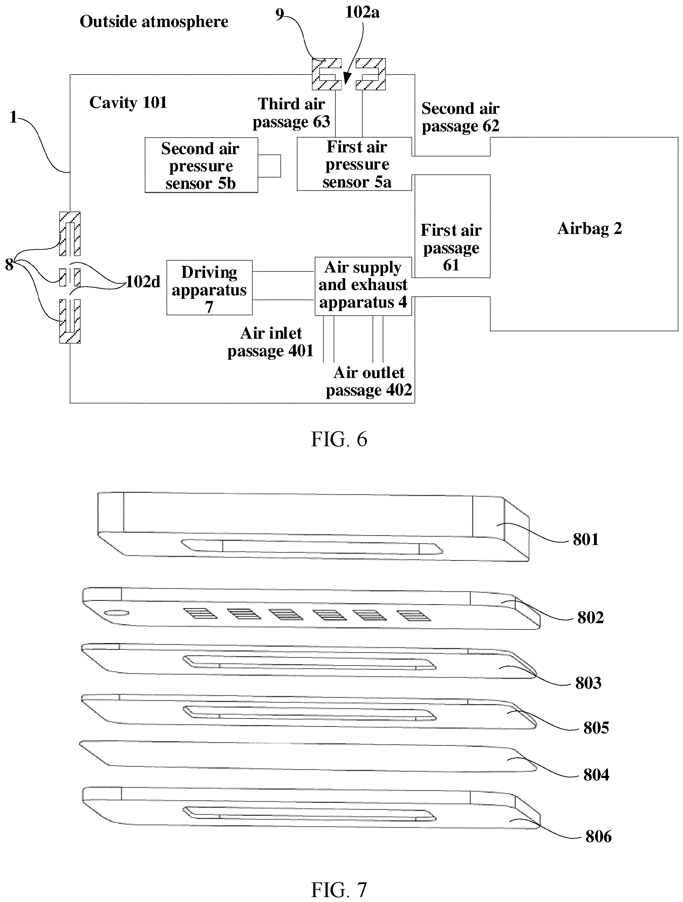

Based on this, refer to FIG. 6. In an embodiment of this application, to reduce the negative pressure in the cavity 101 of the main body 1, the vent hole 102d may be further disposed on the side wall of the main body 1. The vent hole 102d may be configured to communicate the cavity 101 of the main body 1 with the outside atmosphere, to supplement air for the cavity 101 in time, thereby reducing the negative pressure in the cavity 101 of the main body 1, and ensuring a normal operation of functional modules and components in the main body 1. In this application, a specific disposing position of the vent hole 102d is not limited. The vent hole 102d may be adjusted based on a structural design of the main body 1. Further, the vent hole 102a and the vent hole 102d may be provided on a same side wall of the main body 1, or may be provided on different side walls.

To supplement air for the cavity 101 in time, in some possible embodiments of this application, an aperture of the vent hole 102d may be set to be large, for example, may be greater than or equal to 1 mm. In addition, there may be a plurality of vent holes 102d, and the plurality of vent holes 102d may be arranged in an array but are not limited to be arranged in an array.

In this embodiment shown in FIG. 6, to make the entire blood pressure measuring device has good waterproofness, a first waterproof ventilation apparatus 8 may be disposed at the vent hole 102d. The first waterproof ventilation apparatus 8 not only has a waterproof characteristic, but also can enable air in the outside atmosphere to pass through, to enter the cavity 101 of the main body 1. FIG. 7 is a schematic diagram of a structure of a first waterproof ventilation apparatus 8 according to an embodiment of this application. The first waterproof ventilation apparatus 8 may include a plurality of layer structures disposed in stacks, for example, including foam 801, a balance hole steel sheet 802, a polyethylene terephthalate (polyethylene terephthalate, PET) layer, and a waterproof film 804. The balance hole steel sheet 802 is disposed between the foam 801 and a PET layer 803. The foam 801 and the PET layer 803 may be separately bonded and fixed to the balance hole steel sheet 802. Since strength of the balance hole steel sheet 802 is high, the balance hole steel sheet can support the entire waterproof ventilation apparatus. In addition, the waterproof film 804 may be disposed on a side of the PET layer 803 that is away from the balance hole steel sheet 802. The waterproof film 804 may be bonded and fixed to the PET layer 803 through a double-sided tape 805. This is not limited. When the waterproof ventilation apparatus is disposed on the main body 1, a side of the waterproof film 804 of the first waterproof ventilation apparatus 8 that is away from the PET layer 803 may be bonded and fixed to the side wall of the main body 1 through the waterproof glue 806, and the vent hole 102d is covered.

In addition, to meet a requirement of the blood pressure measuring device for an airflow rate in a blood pressure measuring process, in some embodiments of this application, an air permeating volume of the first waterproof ventilation apparatus 8 may be set. For example, the air permeating volume of the first waterproof ventilation apparatus 8 may be greater than or equal to 100 ml/min. In some other embodiments of this application, a disposing area of the first waterproof ventilation apparatus 8 may be further adjusted. For example, an area of the first waterproof ventilation apparatus 8 may be greater than 10 mm².

Using the blood pressure measuring device provided in this application, air permeability of the entire blood pressure measuring device can be met by providing the vent hole 102d. In addition, the first waterproof ventilation apparatus 8 is disposed at the vent hole 102d, so that the blood pressure measuring device has good waterproofness and air permeability, to enable the blood pressure measuring device to be used in some scenarios that require a high waterproof level, thereby expanding an application scenario of the blood pressure measuring device. In addition, since in a process of performing blood pressure measurement by using the blood pressure measuring device provided in this application, a pressure difference measured by the first air pressure sensor 5a is not affected by the air pressure in the cavity 101 of the main body 1, making a blood pressure value measured by the blood pressure measuring device become accurate.

It can be learned from introductions of the foregoing embodiment that the vent hole 102a of the blood pressure measuring device provided in this embodiment of this application is only configured to communicate a second air hole 502 of the first air pressure sensor 5a with the outside atmosphere. In some scenarios in which a waterproof requirement of the first air pressure sensor 5a is not high, or the first air pressure sensor 5a has a waterproof structure, or an aperture of the vent hole 102a is small, a waterproof design may not be performed on the vent hole 102a, thereby simplifying a structure of the blood pressure measuring device. However, in some blood pressure measuring devices with high waterproof requirements, a waterproof ventilation apparatus may be alternatively disposed at the vent hole 102a. Still refer to FIG. 3. In this embodiment, a second waterproof ventilation apparatus 9 is disposed at the vent hole 102a. The second waterproof ventilation apparatus 9 may be the same as or different from the first waterproof ventilation apparatus 8 at the vent hole 102d. This is not specifically limited in this application.

It should be noted that in this embodiment shown in FIG. 5, both the air inlet passage 401 and the air outlet passage 402 of the air supply and exhaust apparatus 4 directly communicate with the outside atmosphere through the vent hole provided on the side wall of the main body 1. Therefore, in this embodiment shown in FIG. 5, the vent hole 102d for communicating the cavity 101 with the outside atmosphere may not be provided on the side wall of the main body 1, so that the blood pressure measuring device has good waterproofness. In addition, since the vent hole 102b and the vent hole 102c are configured for intake and exhaust of the air supply and exhaust apparatus 4, sizes of the vent hole 102b and the vent hole 102c may be small. Waterproof ventilation apparatuses may not be disposed at the vent hole 102b and the vent hole 102c. However, in some blood pressure measuring devices with high waterproof requirements, the waterproof ventilation apparatuses may be alternatively disposed at the vent hole 102b and the vent hole 102c. The waterproof ventilation apparatuses may be disposed with reference to the foregoing first waterproof ventilation apparatus 8 and the foregoing second waterproof ventilation apparatus 9. Details are not described here again.

Since in this embodiment shown in FIG. 6, the air inside the cavity 101 of the main body 1 is mainly supplemented through the vent hole 102d, and the first waterproof ventilation apparatus 8 is provided at the vent hole 102d. If a problem such as blockage occurs in the first waterproof ventilation apparatus 8, air permeability of the first waterproof ventilation apparatus 8 is greatly reduced, thereby causing an impact of the entire blood pressure measuring device on accuracy of the blood pressure measurement. To resolve this problem, still refer to FIG. 6. A second air pressure sensor 5b may be alternatively disposed in the cavity 101 of the main body 1 of the blood pressure measuring device. The second air pressure sensor 5b is an absolute pressure sensor. Assuming that air pressure in the cavity 101 of the main body 1 is P 1, an air pressure value measured by the second air pressure sensor 5b is P1.

In this application, a first threshold may be set for P1. In this way, when the air pressure value P1 measured by the second air pressure sensor 5b is less than the set first threshold, it is determined that the air permeability of the first waterproof ventilation apparatus 8 is good. In this embodiment of this application, a value of the first threshold may be set based on a specific application scenario. For example, the first threshold may be set to 95 KPa. In this way, when the air pressure value P1 measured by the second air pressure sensor 5b is greater than 95 KPa, it is considered that air permeability of the first waterproof ventilation apparatus 8 is good.

In addition, when the air pressure value P1 measured by the second air pressure sensor 5b is lower than the first threshold, it is determined that the first waterproof ventilation apparatus 8 is blocked. In this case, a user may replace or clean the first waterproof ventilation apparatus 8 to ensure security of the blood pressure measuring apparatus and stability of the blood pressure measurement.

It may be understood that in this application, the first waterproof ventilation apparatus 8 may be disposed outside the main body 1, or may be disposed inside the main body 1. In addition, the first waterproof ventilation apparatus 8 and the main body 1 may be fixed in a manner of thread locking, clamping, or the like, to implement a detachable connection between the first waterproof ventilation apparatus 8 and the main body 1, thereby facilitating replacement, cleaning, and the like of the first waterproof ventilation apparatus 8.

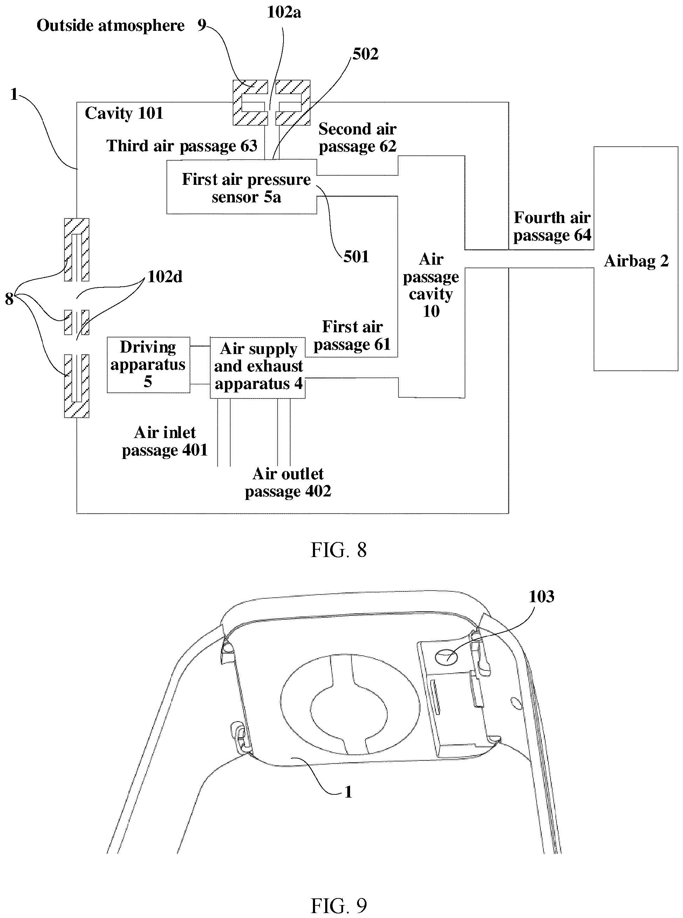

FIG. 8 is a schematic diagram of a framework structure of a blood pressure measuring device according to another possible embodiment of this application. A difference between the blood pressure measuring device in this embodiment shown in FIG. 8 and the blood pressure measuring device in this embodiment shown in FIG. 6 mainly lies in: In this embodiment shown in FIG. 8, the blood pressure measuring device further includes an air passage cavity 10. The air passage cavity 10 is disposed in the cavity 101 of the main body 1. In addition, the air passage cavity 10 may be fixed to a side of the side wall of the main body 1 that faces the cavity 101 in a manner of bonding or threaded connection, to improve structural stability of the air passage cavity 10.

Based on the foregoing change of a structure of the blood pressure measuring device, in this embodiment of this application, a connection manner between a first air pressure sensor 5a, an air supply and exhaust apparatus 4, and an airbag 2 is also adaptively changed. During a specific implementation, still refer to FIG. 8. The air supply and exhaust apparatus 4 is communicated with the air passage cavity 10 through a first air passage 61. The first air hole 501 of the first air pressure sensor 5a is communicated with the air passage cavity 10 through a second air passage 62. In this way, the first air passage 61 may be communicated with the second air passage 62 through the air passage cavity 10. In addition, the air passage cavity 10 may be communicated with the air cavity of the airbag 2 through a fourth air passage 64. Another structure of the blood pressure measuring device in this embodiment shown in FIG. 8 may be disposed with reference to any one of the foregoing embodiments. Details are not described here again.

Using the blood pressure measuring device provided in this embodiment of this application, by adding a design of the air passage cavity 10, an air inlet passage 401 and an air outlet passage 402 of the air supply and exhaust apparatus 4 may be communicated with the air cavity of the airbag 2 through the air passage cavity 10. When the air supply and exhaust apparatus 4 operates, the air supply and exhaust apparatus 4 may draw gas from the cavity 101 of the main body 1 into the air passage cavity 10, and then the gas enters the air cavity of the airbag 2. In addition, the air supply and exhaust apparatus 4 may exhaust the gas in the airbag 2 by exhausting the gas in the air passage cavity 10 through the air outlet passage 402.

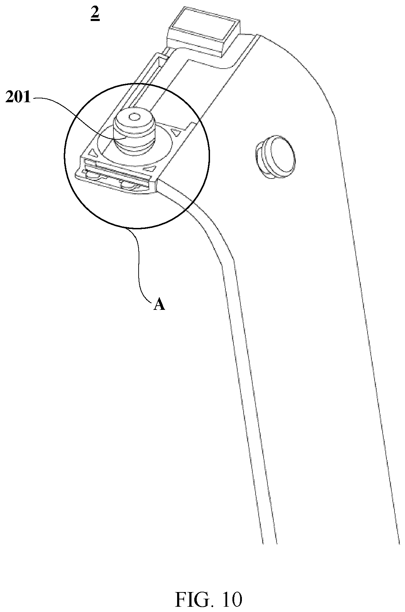

In addition, after the first air pressure sensor 5a is communicated with the air supply and exhaust apparatus 4 through the air passage cavity 10, the first air pressure sensor 5a may be communicated with the airbag 2 through only one air passage (the fourth air passage 64). FIG. 9 is a schematic diagram of a structure of the main body 1 of the blood pressure measuring device corresponding to FIG. 8 according to a possible embodiment of this application. It may be learned from FIG. 9 that only one connection hole 103 for connecting to the airbag 2 may be provided on a side wall of the main body 1 of the blood pressure measuring device. In addition, FIG. 10 is a schematic diagram of a structure of the airbag 2 of the blood pressure measuring device corresponding to FIG. 8 according to a possible embodiment of this application. In this embodiment, an air nozzle 201 may be provided in the airbag 2. The air nozzle 201 may be inserted into an opening of the main body 1 shown in FIG. 9, to implement connection between the airbag 2 and a single air nozzle of the main body 1. This can effectively reduce a quantity of connection holes 103 provided on the entire blood pressure measuring device, improve overall sealing of the blood pressure measuring device, and reduce a risk of damages to functional modules and components in the cavity 101 of the main body 1 of the blood pressure measuring device, thereby prolonging a service life of the blood pressure measuring device.

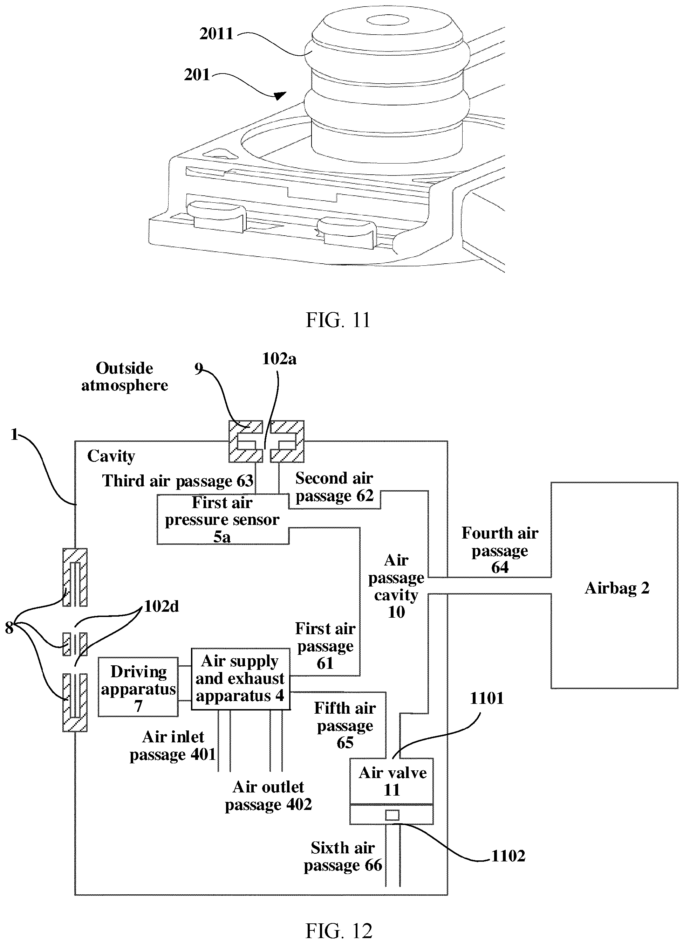

In addition, in this embodiment of this application, a specific structure of the air nozzle 201 of the airbag 2 is not limited. For example, FIG. 11 is a schematic diagram of a structure of the air nozzle 201 of the airbag 2 according to a possible embodiment of this application. The air nozzle 201 may protrude from a side surface of the airbag 2 in a direction toward the main body 1 and protrude from the airbag 2. A clamping structure 2011 may be disposed on the air nozzle 201. The clamping structure 2011 may be, but not limited to, an annular convex structure protruding from the surface of the air nozzle 201. In this way, when the air nozzle 201 is inserted into the connection hole 103, the air nozzle 201 may be clamped to the connection hole 103 of the main body 1 through the clamping structure 2011. In addition, based on a reasonable design, the clamping structure 2011 can further play a waterproof and sealing role. In some possible embodiments of this application, the air nozzle 201 may be alternatively disposed on the main body 1, and the connection hole 103 may be provided in the airbag 2. In this case, a connection between the main body 1 and the airbag 2 may also be implemented by inserting the air nozzle and the connection hole 103.

It may be understood that when the airbag 2 is connected to the main body 1 through the single air nozzle, an air passage cavity 10 may be communicated with the air nozzle 201 of the airbag 2 through a fourth air passage 64, to implement communication between the air passage cavity 10 and the air cavity of the airbag 2.

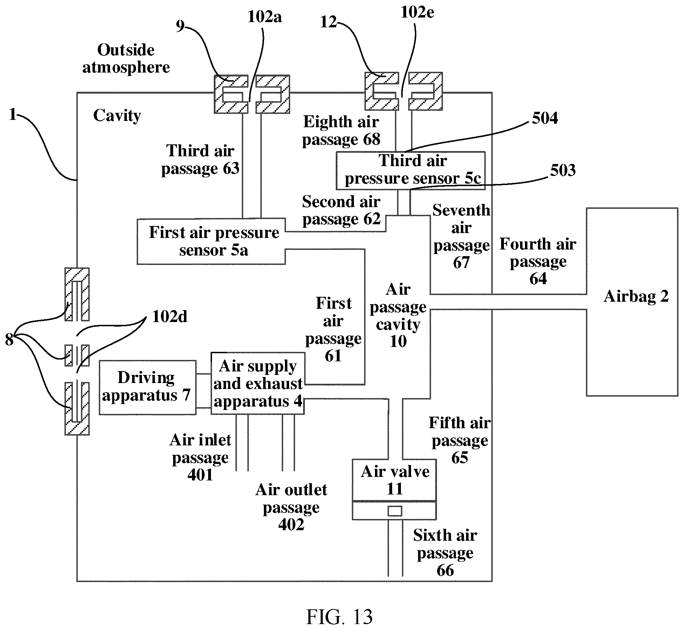

It should be noted that another structure of the blood pressure measuring device shown in FIG. 8 may be disposed with reference to any one of the foregoing embodiments. For example, when the vent hole 102b and the first waterproof ventilation apparatus 8 are disposed on the side wall of the main body 1 of the blood pressure measuring device shown in FIG. 8, the second air pressure sensor 5b may be alternatively disposed in the cavity 101 of the blood pressure measuring device shown in FIG. 8, to measure air permeability of the first waterproof ventilation apparatus. For another example, protrusions may be disposed in pipes of the first air passage 61, the second air passage 62, and a third air passage 63. A specific configuration of another structure of the blood pressure measuring device shown in FIG. 13 is not described one by one here.

In some embodiments of this application, to ensure security of the blood pressure measuring device, an air valve 11 may be further disposed in the blood pressure measuring device. The air valve 11 may be communicated with the air supply and exhaust apparatus 4, and may be used as a spare air outlet port of the air supply and exhaust apparatus 4. During a specific implementation, FIG. 12 is a schematic diagram of a framework structure of a blood pressure measuring device according to another possible embodiment of this application. By comparing the blood pressure measuring device shown in FIG. 12 with the blood pressure measuring device shown in FIG. 8, it can be learned that a difference between the two embodiments mainly lies in: An air valve 11 is further disposed in the blood pressure measuring device in this embodiment shown in FIG. 12. The air valve 11 includes two air openings, and the two air openings may be defined as a first air opening 1101 and a second air opening 1102 respectively. The first air opening 1101 may be communicated with the air passage cavity 10 through a fifth air passage 65, and the second air opening 1102 may be communicated with the cavity 101 of the main body 1 through a sixth air passage 66.

In addition, it can be learned from foregoing introductions that the air inlet passage 401 and the air outlet passage 402 of the air supply and exhaust apparatus 4 directly communicate with outside atmosphere through a vent hole on the side wall of the main body 1. In some embodiments of this application, the second air opening 1102 of the air valve 11 may be alternatively enabled to directly communicate with the outside atmosphere through a sixth air passage 66 and the vent hole provided on the side wall of the main body 1, to reduce the impact of operation of the air valve 11 on the air pressure in the cavity 101 of the main body 1.

In this embodiment of this application, since the air outlet passage 402 of the air supply and exhaust apparatus 4 is communicated with the air passage cavity 10, the air valve 11 may be communicated with the air outlet passage 402 of the air supply and exhaust apparatus 4. In this way, in a case of a failure of the air outlet passage 402 of the air supply and exhaust apparatus 4, a blockage of the air outlet passage 402, or the like, exhaust of the air passage cavity 10 can be implemented by opening the air valve 11, thereby avoiding damages to components such as the air supply and exhaust apparatus 4, the first air pressure sensor 5a, or the airbag 2, to ensure security of the blood pressure measuring device in a process of blood pressure measurement.

In this application, a specific disposing form of the air valve 11 is not limited. For example, the air valve 11 may be a solenoid valve. In this way, the opening and closing of the air valve 11 may be programmed as required, thereby simplifying operations of the blood pressure measuring device and improving user experience. For example, in a possible embodiment of this application, the air valve 11 may be configured as: When air pressure in the air passage cavity 10 is greater than a particular value (for example, 300 mmHg), the air valve 11 is electrically controlled to be opened to deflate. When the air pressure value in the air passage cavity 10 is reduced to a particular value (for example, 10 mmHg) or below, the air valve 11 is closed. Therefore, the air valve 11 can be adaptively opened or closed based on the air pressure value in the air passage cavity 10, so that the air pressure in the entire air passage system is maintained in a stable state, to improve operating reliability of the blood pressure measuring device.

It can be learned from introductions of the air valve 11 in the foregoing embodiment that security performance of the blood pressure measuring device can be effectively improved by disposing the air valve 11. Based on this, in some possible embodiments of this application, the air valve 11 may be alternatively disposed on the air passage of the blood pressure measuring device (for example, the first air passage 61, the third air passage 63, or a fourth air passage 64), to control opening and disclosing of a corresponding air passage through the air valve 11, to implement flexible control on a flow condition of gas in the air passage, and further improve operating reliability of the blood pressure measuring device.

It should be noted that another structure of the blood pressure measuring device shown in FIG. 12 may be disposed with reference to any one of the foregoing embodiments. For example, when a vent hole 102d and the first waterproof ventilation apparatus 8 are disposed on the side wall of the main body 1 of the blood pressure measuring device shown in FIG. 12, the second air pressure sensor 5b may be alternatively disposed in the cavity 101 of the blood pressure measuring device shown in FIG. 12, to measure air permeability of the first waterproof ventilation apparatus. For another example, protrusions may be disposed in pipes of the first air passage 61, the second air passage 62, the third air passage 63, and the fourth air passage 64. A specific configuration of another structure of the blood pressure measuring device shown in FIG. 12 is not described one by one here.

In addition, in this application, to improve accuracy of measuring a blood pressure value by the blood pressure measuring device, a calibration device may be added to the first air pressure sensor 5a, to calibrate an air pressure value in the air cavity of the airbag 2 measured by the first air pressure sensor 5a. During a specific implementation, FIG. 13 is a schematic diagram of a framework structure of a blood pressure measuring device according to another possible embodiment of this application. In this embodiment, a third air pressure sensor 5c is added to the blood pressure measuring device. In this embodiment, the third air pressure sensor 5c may also be a differential air pressure sensor. The third air pressure sensor 5c includes a third air hole 503 and a fourth air hole 504. The third air hole 503 may be communicated with the air passage cavity 10 through a seventh air passage 67, to implement communication between the third air hole 503 and the air cavity of the airbag 2. In addition, a vent hole 102e is provided on the side wall of the main body 1. The fourth air hole 504 of the third air pressure sensor 5c is communicated with the outside atmosphere through an eighth air passage 68. In this application, a specific disposing position of the vent hole 102e is not limited. The vent hole 102e may be adjusted based on a structural design of the main body 1 and a specific disposing position of the third air pressure sensor 5c. In addition, it may be understood that a third waterproof ventilation apparatus 12 may be disposed at the vent hole 102e. The third waterproof ventilation apparatus 12 may be disposed with reference to introductions of the first waterproof ventilation apparatus 8 at the vent hole 102d in any one of the foregoing embodiments. Details are not described here again.

It can be understood from the foregoing introductions of a specific configuration of the third air pressure sensor 5c that both the third air pressure sensor 5c and the first air pressure sensor 5a measure a pressure difference between the air passage cavity 10 and the outside atmosphere. Based on this, whether the measurement of the first air pressure sensor 5a is accurate may be determined by comparing a pressure difference measured by the first air pressure sensor 5a with a pressure difference measured by the third air pressure sensor 5c. The determining process may be, for example, as follows: When a difference between a pressure difference measured by the first air pressure sensor 5a and a pressure difference measured by the third air pressure sensor 5c is within a first threshold range, it is determined that the pressure difference measured by the first air pressure sensor 5a is accurate. In addition, the blood pressure measuring device is enabled to calculate a corresponding blood pressure value based on the pressure difference. In this application, the first threshold range may be set based on a specific application scenario. For example, the first threshold range may be -200 pa to 200 pa.

In addition, when a difference between a pressure difference measured by the first air pressure sensor 5a and a pressure difference measured by the third air pressure sensor 5c is outside the first threshold range, it is determined that the pressure difference measured by the first air pressure sensor 5a is inaccurate. A group of differential pressure data measured by the first air pressure sensor is discarded, and a next measurement is performed until a difference between a pressure difference measured by the first air pressure sensor 5a and a pressure difference measured by the third air pressure sensor 5c falls within the first threshold range. In addition, the blood pressure measuring device is enabled to calculate a corresponding blood pressure value based on the pressure difference.

It should be noted that another structure of the blood pressure measuring device shown in this embodiment in FIG. 13 may be disposed with reference to any one of the foregoing embodiments. For example, when the vent hole 102d and the first waterproof ventilation apparatus 8 are disposed on the side wall of the main body 1 of the blood pressure measuring device shown in FIG. 13, the second air pressure sensor 5b may be alternatively disposed in the cavity 101 of the blood pressure measuring device shown in FIG. 13, to measure air permeability of the first waterproof ventilation apparatus 8. For another example, protrusions may be provided in pipes of the first air passage 61, the second air passage 62, the third air passage 63, the fourth air passage 64, the fifth air passage 65, the sixth air passage 66, the seventh air passage 67, and the eighth air passage 68. For another example, when the air passage cavity 10 is not disposed in the blood pressure measuring device, the third air hole 503 of the third air pressure sensor 5c may directly communicate with the air cavity of the airbag 2 through the seventh air passage 67. A specific configuration of another structure of the blood pressure measuring device shown in FIG. 13 is not described one by one here.

Using the blood pressure measuring device in this embodiment of this application, the third air pressure sensor 5c is added to calibrate the pressure difference measured by the first air pressure sensor 5a. This can effectively improve accuracy of measuring a blood pressure value by the blood pressure measuring device.

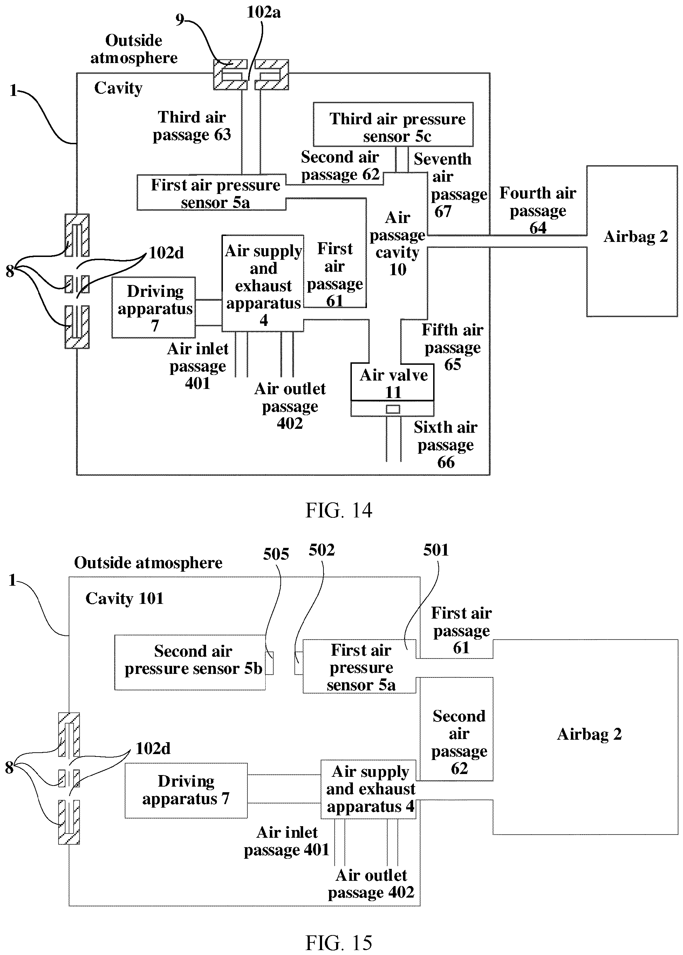

In this application, in addition to using the differential air pressure sensor shown in FIG. 13, the third air pressure sensor 5c may alternatively use an absolute air pressure sensor. During a specific implementation, FIG. 14 is a schematic diagram of a framework structure of a blood pressure measuring device according to another possible embodiment of this application. In this embodiment, the third air pressure sensor 5c has only one third air hole. The third air hole is communicated with the air passage cavity 10 through the seventh air passage 67.

In this embodiment shown in FIG. 14, air pressure in the air cavity of the airbag 2 measured by the first air pressure sensor 5a may be calibrated by air pressure in the airbag 2 measured by the absolute third air pressure sensor 5c. A calibration method may be, for example, as follows: When a difference between the air pressure in the air cavity of the airbag 2 measured by the first air pressure sensor 5a and the air pressure in the air cavity of the airbag 2 measured by the third air pressure sensor 5c is within a first threshold range, it is determined that the air pressure in the air cavity of the airbag 2 measured by the first air pressure sensor 5a is accurate. In addition, the blood pressure measuring device is enabled to calculate a corresponding blood pressure value based on the air pressure. In this application, the first threshold range may be set based on a specific application scenario. For example, the first threshold range may be -200 pa to 200 pa.

In addition, when a difference between the air pressure in the air cavity of the airbag 2 measured by the first air pressure sensor 5a and the air pressure in the air cavity of the airbag 2 measured by the third air pressure sensor 5c is outside the first threshold range, it is determined that the air pressure in the air cavity of the airbag 2 measured by the first air pressure sensor is inaccurate. A group of air pressure data measured by the first air pressure sensor 5a is discarded, and a next measurement is performed until a difference between the air pressure in the air cavity of the airbag 2 measured by the first air pressure sensor 5a and the air pressure in the air cavity of the airbag 2 measured by the third air pressure sensor 5c falls within the first threshold range. In addition, the blood pressure measuring device is enabled to calculate a corresponding blood pressure value based on the air pressure.

It can be learned from introductions of an operating principle of the first air pressure sensor 5a in the foregoing embodiment that the air pressure difference measured by the first air pressure sensor 5a is directly determined through external atmospheric pressure and the air pressure in the air cavity of the airbag 2. Generally, the external atmospheric pressure is considered to be a constant value. Therefore, the air pressure value in the air cavity of the airbag 2 can be directly obtained through the pressure difference measured by the first air pressure sensor 5a and the external atmospheric pressure.

It should be noted that another structure of the blood pressure measuring device shown in this embodiment in FIG. 14 may be disposed with reference to any one of the foregoing embodiments. For example, when the vent hole 102d and the first waterproof ventilation apparatus 8 are disposed on the side wall of the main body 1 of the blood pressure measuring device shown in FIG. 14, the second air pressure sensor 5b may be alternatively disposed in the cavity 101 of the blood pressure measuring device shown in FIG. 14, to measure air permeability of the first waterproof ventilation apparatus 8. For another example, protrusions may be provided in pipes of the first air passage 61, the second air passage 62, the third air passage 63, the fourth air passage 64, the fifth air passage 65, the sixth air passage 66, and the seventh air passage 67. For another example, when the air passage cavity 10 is not disposed in the blood pressure measuring device, the third air hole of the third air pressure sensor 5c may directly communicate with the air cavity of the airbag 2 through the seventh air passage 67. A specific configuration of another structure of the blood pressure measuring device shown in FIG. 14 is not described one by one here.

Using the blood pressure measuring device in this embodiment of this application, the third air pressure sensor 5c is added to calibrate a result measured by the first air pressure sensor 5a, thereby effectively improving accuracy of measuring a blood pressure value by the blood pressure measuring device.

FIG. 15 is a schematic diagram of a structure of a blood pressure measuring device according to another embodiment of this application. A structure of the blood pressure measuring device in this embodiment is different from that in any of the foregoing embodiments, and the difference mainly lies in: The first air pressure sensor 5a is disposed inside the cavity 101 of the main body 1, no vent hole 102a is disposed on the side wall of the main body 1, and the second air hole 502 of the first air pressure sensor 5a is connected to the cavity 101 of the main body 1. In this embodiment, a pressure difference measured by the first air pressure sensor 5a is determined by air pressure in the cavity 101 of the main body 1 and air pressure in the air cavity of the airbag 2.

To reduce impact of a change of the air pressure in the cavity 101 of the main body 1 on accuracy of the air pressure difference measured by the first air pressure sensor 5a, still refer to FIG. 15. A second air pressure sensor 5b may be further disposed in the cavity 101 of the main body 1. The second air pressure sensor 5b is an absolute pressure sensor, and the second air pressure sensor 5b is disposed close to the first air pressure sensor 5a. Only one fifth air hole 505 is provided in the second air pressure sensor 5b. The fifth air hole 505 faces to the second air hole 502 of the first air pressure sensor 5a. In a possible embodiment, the fifth air hole 505 and the second air hole 502 may be enabled to be arranged coaxially. In addition, a distance between the fifth air hole 505 and the second air hole 502 is less than or equal to 1 mm.

Since the second air pressure sensor 5b includes only one fifth air hole 505, the second air pressure sensor 5b can measure the air pressure in the cavity 101 of the main body 1. In addition, since the distance between the fifth air hole 505 and the second air hole 502 is small, air pressure measured on a side of the fifth air hole 505 may be considered equal to air pressure measured on a side of the second air hole 502.

When using the blood pressure measuring device shown in FIG. 15 of this application to perform blood pressure measurement and when the air cavity of the airbag 2 is inflated by the air supply and exhaust apparatus 4, air in the cavity 101 of the main body 1 is drawn into the air cavity of the airbag 2. In this way, low air pressure space lower than atmospheric pressure is formed in the cavity 101 of the main body 1. Assuming that the air pressure in the cavity 101 of the main body 1 is P1 (a read value of the absolute pressure sensor), the external atmospheric pressure is P0, and the air pressure in the air cavity of the airbag 2 is P. It may be understood that the air pressure P is a differential pressure value in the air cavity of the airbag 2, and an absolute pressure value of the air cavity of the airbag 2 is P+P0. A relationship between the foregoing air pressure values is as follows: A read value of the first air pressure sensor 5a is P2=P+P0-P1. Therefore, actual air pressure in the airbag 2 is P=P2-ΔP (ΔP=P0-P1), then P=P2- (P0-P1).

It may be understood that in this embodiment of this application, although the air pressure in the main body 1 is low, the second air pressure sensor 5b is disposed in the cavity 101 of the main body 1. In this way, a difference between the air pressure P0 of the outside atmosphere and the air pressure value measured by the second air pressure sensor 5b may be used as an error value for calculating the air pressure in the air cavity of the airbag 2, and an actual air pressure value in the airbag 2 may be obtained. Therefore, blood pressure measurement accuracy of the blood pressure measuring device can be effectively improved.

In addition, in this embodiment of this application, the first waterproof ventilation apparatus 8 may be disposed at the vent hole 102d of the blood pressure measuring device. In this way, while the entire blood pressure measuring device is waterproofed, accuracy of a blood pressure value measured by the blood pressure measuring device can be ensured.

Since the second air pressure sensor 5b is disposed in the cavity 101 of the main body 1, and the second air pressure sensor 5b is an absolute pressure sensor. It can be understood from the foregoing introductions of measuring a blockage degree of the first waterproof ventilation apparatus 8 by disposing the second air pressure sensor 5b, in this embodiment shown in FIG. 15 of this application, based on a process of blood pressure measurement, the blockage degree of the first waterproof ventilation apparatus 8 is determined by the air pressure value P1 measured by the second air pressure sensor 5b. During a specific implementation, a first threshold may be set for P1. When the air pressure value P 1 measured by the second air pressure sensor 5b is greater than the first threshold, it is determined that air permeability of the first waterproof ventilation apparatus 8 is good, and/or when the air pressure value P1 measured by the second air pressure sensor 5b is less than the first threshold, it is determined that the air permeability of the first waterproof ventilation apparatus 8 is poor. In this case, a user may replace or clean the first waterproof ventilation apparatus 8, to ensure security of the blood pressure measuring device and stability of blood pressure measurement, so that a blood pressure value measured by the blood pressure measuring device is accurate.

In this application, the first waterproof ventilation apparatus 8 may be disposed outside the main body 1, or may be disposed inside the main body 1. In addition, the first waterproof ventilation apparatus 8 and the main body 1 may be fixed in a manner of thread locking, clamping, or the like, to implement a detachable connection between the first waterproof ventilation apparatus 8 and the main body 1, thereby facilitating replacement, cleaning, and the like of the first waterproof ventilation apparatus 8.

It should be noted that another structure of the blood pressure measuring device in this embodiment shown in FIG. 15 may be disposed with reference to any one of the foregoing embodiments. For example, the air passage cavity 10 may be disposed in the blood pressure measuring device, to enable the air supply and exhaust apparatus 4 to be communicated with the air passage cavity 10 through the first air passage 61, enable to the first air hole 501 of the first air pressure sensor 5a to be communicated with the air passage cavity 10 through the second air passage 62, and enable the air passage cavity 10 to be communicated with the airbag 2 through the fourth air passage 64, so that a connection between the main body 1 and a single air nozzle of the airbag 2 is implemented. A specific configuration of another structure of the blood pressure measuring device shown in FIG. 15 is not described one by one here.

When using the blood pressure measuring device provided in this application to perform blood pressure measurement, impact of the air pressure in the cavity 101 of the main body 1 of the blood pressure measuring device on a measurement value of the first air pressure sensor 5a can be effectively reduced, so that a blood pressure measuring result is accurate. In addition, the first waterproof ventilation apparatus 8 may be disposed on the main body 1 of the blood pressure measuring device, to implement a waterproof design of the blood pressure measuring device. In addition, the air permeability of the first waterproof ventilation apparatus 8 can be measured by disposing the second air pressure sensor 5b, to prevent the blockage of the first waterproof ventilation apparatus 8 from affecting the measurement result of the blood pressure measuring device.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A blood pressure measuring device, comprising: a main body, an airbag, an air supply and exhaust apparatus, and a first air pressure sensor, wherein
the main body comprises a cavity, and the cavity is enclosed by a plurality of side walls; the air supply and exhaust apparatus and the first air pressure sensor are disposed in the cavity;
the airbag has an air cavity, and the airbag is fixed to an end of the main body;
the air supply and exhaust apparatus comprises an air inlet passage and an air outlet passage, and the air supply and exhaust apparatus is communicated with the air cavity of the airbag through a first air passage; and
the first air pressure sensor comprises a first air hole, a second air hole, and a pressure film, and the pressure film is located between the first air hole and the second air hole; and a first vent hole is provided on the side wall, the first air hole is communicated with the air cavity of the airbag through a second air passage, and the second air hole is communicated with the first vent hole through a third air passage, so that the first air pressure sensor is communicated with outside atmosphere through the first vent hole.

2. The blood pressure measuring device according to claim 1, wherein a second vent hole is further provided on the side wall, and a first waterproof ventilation apparatus is disposed at the second vent hole.

3. The blood pressure measuring device according to claim 2, wherein an air permeating volume of the first waterproof ventilation apparatus is greater than or equal to 100 ml/min.

4. The blood pressure measuring device according to claim 2 or 3, wherein the blood pressure measuring device further comprises a second air pressure sensor, the second air pressure sensor is disposed in the cavity of the main body, and the blood pressure measuring device further determines air permeability of the first waterproof ventilation apparatus based on an air pressure value measured by the second air pressure sensor.

5. The blood pressure measuring device according to claim 4, wherein the second air pressure sensor is an absolute pressure sensor;
when the air pressure value measured by the second air pressure sensor is greater than a first threshold, it is determined that the air permeability of the first waterproof ventilation apparatus is good; and/or
when the air pressure value measured by the second air pressure sensor is lower than the first threshold, it is determined that the first waterproof ventilation apparatus is blocked.

6. The blood pressure measuring device according to any one of claims 1 to 5, wherein the blood pressure measuring device further comprises an air passage cavity, and the air passage cavity is disposed in the cavity of the main body; and
the air supply and exhaust apparatus is communicated with the air passage cavity through the first air passage, and the first air hole of the first air pressure sensor is communicated with the air passage cavity through the second air passage; and the air passage cavity is communicated with the air cavity of the airbag through a fourth air passage.

7. The blood pressure measuring device according to claim 6, wherein the blood pressure measuring device further comprises an air valve, the air valve comprises a first air opening and a second air opening, the first air opening is communicated with the air passage cavity through a fifth air passage, and the second air opening is communicated with the cavity of the main body through a sixth air passage.

8. The blood pressure measuring device according to claim 6 or 7, wherein the blood pressure measuring device further comprises a third air pressure sensor, and the third air pressure sensor comprises a third air hole and a fourth air hole; and a third vent hole is further provided on the side wall of the main body, the third air hole is communicated with the air passage cavity through a seventh air passage, and the fourth air hole is communicated with the third vent hole through an eighth air passage.

9. The blood pressure measuring device according to claim 8, wherein when a difference between a pressure difference measured by the first air pressure sensor and a pressure difference measured by the third air pressure sensor falls within a first threshold range, it is determined that the pressure difference measured by the first air pressure sensor is accurate; and/or
when a difference between a pressure difference measured by the first air pressure sensor and a pressure difference measured by the third air pressure sensor falls outside the first threshold range, it is determined that the pressure difference measured by the first air pressure sensor is inaccurate.

10. The blood pressure measuring device according to claim 6 or 7, wherein the blood pressure measuring device further comprises a third air pressure sensor, and the third air pressure sensor is an absolute air pressure sensor; and the third air pressure sensor comprises a third air hole, and the third air hole is communicated with the air passage cavity through a seventh air passage.

11. The blood pressure measuring device according to claim 10, wherein when a difference between air pressure in the airbag measured by the first air pressure sensor and air pressure in the airbag measured by the third air pressure sensor falls within a first threshold range, it is determined that the air pressure in the airbag measured by the first air pressure sensor is accurate; and/or
when a difference between air pressure in the airbag measured by the first air pressure sensor and air pressure in the airbag measured by the third air pressure sensor falls outside the first threshold range, it is determined that the air pressure in the airbag measured by the first air pressure sensor is inaccurate.

12. The blood pressure measuring device according to any one of claims 6 to 11, wherein a connection hole is provided at an end of the main body, the airbag has an air nozzle, and the air nozzle protrudes from a side surface of the airbag in a direction toward the main body; and the air nozzle is inserted into the connection hole, and the fourth air passage is connected to the air nozzle.

13. The blood pressure measuring device according to any one of claims 1 to 12, wherein a fourth vent hole and a fifth vent hole are further provided on the side wall of the main body, the air inlet passage of the air supply and exhaust apparatus is communicated with the outside atmosphere through the fourth vent hole, and the air outlet passage is communicated with the outside atmosphere through the fifth vent hole.

14. The blood pressure measuring device according to any one of claims 1 to 13, wherein a second waterproof ventilation apparatus is further disposed at the first vent hole.

15. The blood pressure measuring device according to any one of claims 1 to 14, wherein a plurality of protrusions are provided on an inner wall of a pipe of the second air passage, and the plurality of protrusions are alternately arranged at intervals along an extension direction of the pipe.

16. The blood pressure measuring device according to any one of claims 1 to 15, wherein the airbag is detachably connected to the main body.

17. The blood pressure measuring device according to any one of claims 1 to 16, wherein the blood pressure measuring device further comprises a photoplethysmography PPG module and an ECG measurement module, and the PPG module and the ECG measurement module are disposed on a bottom surface of the main body.

18. A blood pressure measuring device, comprising: a main body, an airbag, an air supply and exhaust apparatus, a first air pressure sensor and a second air pressure sensor, wherein
the main body comprises a cavity, and the cavity is enclosed by a plurality of side walls; the air supply and exhaust apparatus, the first air pressure sensor, and the second air pressure sensor are disposed in the cavity;
the airbag has an air cavity, and the airbag is fixed to an end of the main body;
the air supply and exhaust apparatus comprises an air inlet passage and an air outlet passage, and the air supply and exhaust apparatus is communicated with the air cavity of the airbag through a first air passage;
the first air pressure sensor comprises a first air hole, a second air hole, and a pressure film, and the pressure film is located between the first air hole and the second air hole; and a first vent hole is provided on the side wall, the first air hole is communicated with the air cavity of the airbag through a second air passage, and the second air hole is communicated with the cavity; and
the second air pressure sensor is an absolute air pressure sensor, the second air pressure sensor comprises a fifth air hole, and the fifth air hole faces to the second air hole.

19. The blood pressure measuring device according to claim 18, wherein the blood pressure measuring device obtains an air pressure value in the air cavity of the airbag based on external atmospheric pressure, an air pressure value measured by the second air pressure sensor, and a pressure difference measured by the first air pressure sensor.

20. The blood pressure measuring device according to claim 18 or 19, wherein the fifth air hole and the second air hole are arranged coaxially.

21. The blood pressure measuring device according to claim 20, wherein a distance between the fifth air hole and the second air hole is less than or equal to 1 mm.

22. The blood pressure measuring device according to any one of claims 18 to 21, wherein a vent hole is further provided on the side wall, and a waterproof ventilation apparatus is disposed at the vent hole; and when the air pressure value measured by the second air pressure sensor is greater than a first threshold, it is determined that air permeability of the waterproof ventilation apparatus is good; and/or
when the air pressure value measured by the second air pressure sensor is lower than the first threshold, it is determined that the waterproof ventilation apparatus is blocked.

23. A blood pressure measuring device, comprising: a main body, an airbag, an air supply and exhaust apparatus, a first air pressure sensor, a second air pressure sensor, and a waterproof ventilation apparatus, wherein
the main body comprises a cavity, and the cavity is enclosed by a plurality of side walls; the air supply and exhaust apparatus, the first air pressure sensor, and the second air pressure sensor are disposed in the cavity;
a vent hole is provided on the side wall, and the waterproof ventilation apparatus covers the vent hole;
the airbag has an air cavity, and the airbag is fixed to an end of the main body;
the first air pressure sensor is configured to measure air pressure in the air cavity of the airbag;
the air supply and exhaust apparatus comprises an air inlet passage and an air outlet passage, and the air supply and exhaust apparatus is communicated with the air cavity of the airbag through a first air passage; and
the second air pressure sensor is an absolute air pressure sensor, and when an air pressure value measured by the second air pressure sensor is greater than a first threshold, it is determined that air permeability of the waterproof ventilation apparatus is good; and/or when the air pressure value measured by the second air pressure sensor is lower than the first threshold, it is determined that the waterproof ventilation apparatus is blocked.
